# EUROPÄISCHE PATENTANMELDUNG

(11) **EP 4 810 470 A1**
(43) Veröffentlichungstag der Anmeldung: **23.09.2026**
(21) Anmeldenummer: 25164642.8
(22) Anmeldetag: 19.03.2025
(51) Int. Cl.: C07C 45/50, C07C 47/02, C07C 47/21, C07F 9/6574, C07F 15/00

(54) **LIGANDEN AUF BASIS VON PHOSPHITEN SOWIE DEREN VERWENDUNG**

(71) Anmelder: Evonik Oxeno GmbH & Co. KG, 45772 Marl (DE)
(72) Erfinder: FRANKE, Robert, 45772 Marl (DE); BÜKER, Julia, 44803 Bochum (DE); SALE, Anna Chiara, 40474 Düsseldorf (DE); BÖRNER, Armin, 18059 Rostock (DE); Holz, Jens, 18196 Kessin (DE); ROMEIKE, Kerstin, 18109 Rostock (DE); WENZEL, Gudrun, 18196 Kessin (DE)
(74) Vertreter: Evonik Patent Association

(57) **Zusammenfassung**

Die vorliegende Erfindung betrifft das technische Gebiet der homogenen Katalyse. Insbesondere betrifft die vorliegende Erfindung Verbindungen bzw. Liganden auf Basis von Phosphor-(III)-Verbindungen und deren Herstellung und Verwendung in Hydroformylierungsreaktionen sowie ein entsprechendes Verfahren zur Hydroformylierung.

## Beschreibung

Die vorliegende Erfindung betrifft das technische Gebiet der homogenen Katalyse. Insbesondere betrifft die vorliegende Erfindung Liganden auf Basis von Phosphor-(III)-Verbindungen sowie deren Herstellung und Verwendung in Hydroformylierungsreaktionen, sowie ein entsprechendes Verfahren zur Hydroformylierung.

Die vorliegende Erfindung betrifft speziell Verbindungen wie in den Ansprüchen 1 bis 5 definiert, ein Verfahren zu deren Herstellung sowie deren Verwendung und ein Verfahren zur Hydroformylierung unter Verwendung der beschriebenen Verbindungen.

Die Reaktion zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Die Produkte der Hydroformylierung werden vielfältig als Lösungsmittel oder als Zwischenprodukte für die Herstellung von Wasch- und Reinigungsmitteln, Schmiermitteln oder Weichmachern für Kunststoffe eingesetzt.

Durch den Einsatz von Katalysatorsystemen mit maßgeschneiderten Liganden, welche die Regio-, Stereo- und Enantioselektivität der Reaktion steuern können, wurde die Hydroformylierung ein wichtiges Werkzeug in der organischen Synthese von Feinchemikalien. In Hydroformylierungsreaktionen werden dabei häufig Verbindungen der Übergangsmetalle der VIII. Gruppe des Periodensystems der Elemente als Katalysatoren verwendet. Bekannte Liganden sind beispielsweise Verbindungen aus den Klassen der Phosphine, Phosphite und Phosphonite mit jeweils dreiwertigem Phosphor P(III). Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

Beispielhafte Liganden, wie sie im Stand der Technik zum Einsatz kommen, sind etwa in der EP 2 947 090 A1 oder der EP 4 059 940 A1 beschrieben.

Eine unaufwändige Zugänglichkeit bzw. Verfügbarkeit und damit verbunden die gute Möglichkeit eines großtechnischen Einsatzes ist ein wichtiges Kriterium für Liganden. Ferner ist das Erreichen hoher Umsätze, gegebenenfalls in Kombination mit einer ausbalancierten n/iso-Selektivität (n/iso = Verhältnis von linearem Aldehyd (= n) zu verzweigtem (= iso) Aldehyd)), insbesondere für die großtechnisches Verwendung, von Bedeutung. Ein weiterer relevanter Aspekt ist das Erreichen von mengeneffizienten Katalysator-zu-Ligandverhältnissen. Hierbei werden speziell möglichst geringe Ligandüberschüsse bei gleichbleibenden Umsätzen bzw. Selektivitäten angestrebt.

Die technische Aufgabe der Erfindung liegt also in der Bereitstellung neuer Verbindungen bzw. Liganden, welche insbesondere großtechnisch in der Hydroformylierung von Olefinen eingesetzt werden können und die Nachteile des Standes der Technik überwinden bzw. zumindest minimieren.

Insbesondere besteht eine Aufgabe der vorliegenden Erfindung darin, gegenüber bekannten Verbindungen bzw. Katalysatorsystemen des Standes der Technik eine gleichwertige oder sogar verbesserte Ausbeute zu liefern, vorzugsweise unter Verwendung möglichst geringer Ligandüberschüsse.

Gegenstand der vorliegenden Erfindung ist eine Verbindung nach Anspruch 1; weitere, vorteilhafte Ausgestaltungen dieses Erfindungsaspekts sind Gegenstand der diesbezüglichen Unteransprüche.

Weiterer Gegenstand der vorliegenden Erfindung - gemäß einem zweiten Aspekt der vorliegenden Erfindung - ist ein Verfahren nach Anspruch 6; weitere, vorteilhafte Ausgestaltungen dieses Erfindungsaspekts sind Gegenstand der diesbezüglichen Unteransprüche.

Wiederum weiterer Gegenstand der vorliegenden Erfindung - gemäß einem dritten Aspekt der vorliegenden Erfindung - ist eine katalytisch aktive Verbindung nach Anspruch 8; weitere, vorteilhafte Ausgestaltungen dieses Erfindungsaspekts sind Gegenstand der diesbezüglichen Unteransprüche.

Schließlich weitere Gegenstände der vorliegenden Erfindung - gemäß einem vierten und fünften Aspekt der vorliegenden Erfindung - sind ein Verfahren zur Hydroformylierung sowie eine diesbezügliche Verwendung; weitere, vorteilhafte Ausgestaltungen dieser Erfindungsaspekte sind Gegenstand der diesbezüglichen Unteransprüche.

Es versteht sich von selbst, dass Besonderheiten, Merkmale, Ausgestaltungen und Ausführungsformen sowie Vorteile oder dergleichen, welche nachfolgend - zu Zwecken der Vermeidung unnötiger Wiederholungen - nur zu einem Erfindungsaspekt ausgeführt werden, selbstverständlich in Bezug auf die übrigen Erfindungsaspekte entsprechend gelten, ohne dass es einer ausdrücklichen Erwähnung bedarf.

Weiterhin gilt, dass alle im Folgenden genannten Werte- bzw. Parameterangaben oder dergleichen grundsätzlich genormt bzw. standardisiert oder explizit angegebenen Bestimmungsverfahren oder mit dem Fachmann auf diesem Gebiet an sich geläufigen Bestimmungsmethoden ermittelt bzw. bestimmt werden können.

Darüber hinaus versteht es sich von selbst, dass alle gewichts- oder mengenbezogenen Prozentangaben vom Fachmann derart ausgewählt werden, dass in der Summe 100 % resultieren.

Dies vorausgeschickt, wird im Folgenden die vorliegende Erfindung näher beschrieben.

Gegenstand der vorliegenden Erfindung - gemäß einem **ersten** Aspekt der vorliegenden Erfindung - ist eine Verbindung gemäß allgemeiner Formel (I) 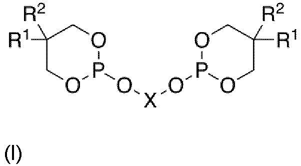
wobei R¹ und R², gleich oder verschieden und/oder unabhängig voneinander, jeweils ausgewählt sind aus einem der folgenden Reste: H und C₁-C₅-Alkyl, insbesondere H, Methyl und *tert-Butyl,* und
wobei X ein zweibindiger organischer Rest, insbesondere ein zweibindiger gesättigter oder ungesättigter, aliphatischer oder aromatischer organischer Rest, ist;
insbesondere wobei die Verbindung bevorzugt symmetrisch, insbesondere punktsymmetrisch oder spiegelsymmetrisch, ausgebildet ist.

Verbindungen gemäß Formel (I) zeichnen sich vorteilhaft durch eine vergleichsweise unkomplizierte synthetische Zugänglichkeit aus. Insbesondere können die Verbindungen auf Grundlage lediglich zweier Vorläufer erhalten werden.

Zudem können Verbindungen gemäß Formel (I) relativ unaufwändig in größeren Mengen hergestellt werden, so dass insbesondere eine großtechnische Verwendung der Verbindungen bzw. ein Einsatz in großtechnischen Verfahren vorteilhaft möglich ist.

Für den bevorzugten Fall, dass Verbindungen gemäß Formel (I) als Liganden in Verfahren zur Hydroformylierung eingesetzt werden, ist es außerdem vorteilhaft möglich, die Verbindungen in relativ geringer Konzentration bzw. in einem vergleichsweise kleinen Verhältnis von Katalysatormetall zu Ligand einzusetzen. Diesbezüglich wirkt sich speziell der bidentate Charakter sowie der symmetrische, insbesondere punktsymmetrische oder spiegelsymmetrische, Aufbau der Verbindungen vorteilhaft aus. Mittels Verbindungen gemäß Formel (I) kann somit ein effizienter Hydroformylierungsprozess umgesetzt werden. Speziell im Vergleich zu monodentaten Liganden kann insgesamt eine deutlich geringere Ligandmenge eingesetzt werden. Dies ist in prozessökonomischer Hinsicht als vorteilhaft zu bewerten.

Auch ist es so, dass Verbindungen gemäß Formel (I) zuverlässig und schnell an das Katalysatormetall koordinieren können, insbesondere wobei die Verbindungen das Katalysatormetall auch einseitig abschirmen.

Verbindungen gemäß Formel (I) weisen außerdem, ohne sich auf diese Theorie beschränken zu wollen, aufgrund der nachfolgend noch eingehender beschriebenen Ausgestaltung des Restes X eine vorteilhafte Kompatibilität mit der durch das Katalysatormetall bevorzugten Ausbildung planarer bzw. oktaedrischer Komplexe auf, was dem Verlauf der Hydroformylierung, insbesondere im Hinblick auf mögliche erzielte Umsätze, insgesamt zuträglich ist.

Die vorgenannten Aspekte können auch für die Orientierung des Verlaufs der Hydroformylierung, insbesondere im Hinblick auf die n/iso-Selektivität der Hydroformylierung (n/iso = Verhältnis von linearem Aldehyd (= n) zu verzweigtem (= iso) Aldehyd)), vorteilhaft sein.

Insgesamt kann mit der vorliegenden Erfindung mittels der Verbindungen gemäß Formel (I) eine mit einer Reihe von Vorteilen versehene Basis für die effiziente Durchführung von Hydroformylierungen unter Realisierung hoher Umsätze und spezifischer Selektivitäten bereitgestellt werden.

Gemäß der vorliegenden Erfindung ist Gegenstand gemäß einem ersten Aspekt insbesondere eine Verbindung gemäß allgemeiner Formel (I) 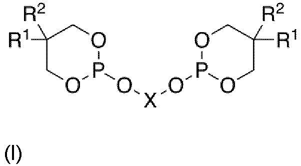
wobei R¹ und R², gleich oder verschieden und/oder unabhängig voneinander, jeweils ausgewählt sind aus einem der folgenden Reste: H und C₁-C₅-Alkyl, insbesondere H, Methyl und *tert-Butyl,* und
wobei X ein zweibindiger organischer Rest, insbesondere ein zweibindiger gesättigter oder ungesättigter, aliphatischer oder aromatischer organischer Rest, ist;
insbesondere wobei die Verbindung bevorzugt symmetrisch, insbesondere punktsymmetrisch oder spiegelsymmetrisch, ausgebildet ist;
vorzugsweise wobei mindestens eine, insbesondere beide, der nachfolgend genannten Verbindungen ausgenommen sind (sog. Disclaimer): 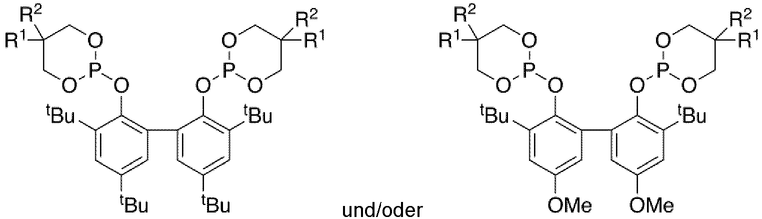
Im Rahmen der vorliegenden Erfindung hat es sich dabei bewährt, wenn X ausgewählt ist aus einem der folgenden Reste:
   - X ist ein aliphatischer Rest gemäß allgemeiner Formel (III) 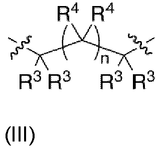
      wobei R³, gleich oder verschieden und/oder unabhängig voneinander, jeweils ausgewählt ist aus einem der folgenden Reste: H und C₁-C₅-Alkyl, insbesondere H, Methyl und *tert*-Butyl,
      wobei R⁴, gleich oder verschieden und/oder unabhängig voneinander, jeweils ausgewählt ist aus einem der folgenden Reste: H und C₁-C₅-Alkyl, insbesondere H, Methyl und *tert*-Butyl, oder wobei zwei Reste R⁴ einen gemeinsamen Ring ausbilden, insbesondere einen gemeinsamen aliphatischen C₅- oder C₆-Ring, und
      wobei n eine ganze Zahl 0, 1, 2 oder 3, insbesondere 1 oder 2, darstellt;
   - X ist ein Rest der allgemeinen Formel (IV) 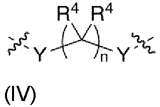
      wobei Y ein gegebenenfalls substituierter aromatischer Rest, insbesondere ein gegebenenfalls substituierter Phenylenrest, vorzugsweise ein gegebenenfalls alkylsubstituierter Phenylenrest, bevorzugt ein gegebenenfalls C₁-C₅-alkylsubstituierter und/oder C₁-C₅-alkoxylsubstituierter Phenylenrest, ist,
      wobei R⁴, gleich oder verschieden und/oder unabhängig voneinander, jeweils ausgewählt ist aus einem der folgenden Reste: H und C₁-C₅-Alkyl, insbesondere H, Methyl und *tert*-Butyl, oder wobei zwei Reste R⁴ einen gemeinsamen Ring ausbilden, insbesondere einen gemeinsamen aliphatischen C₅- oder C₆-Ring, und
      wobei n eine ganze Zahl 0, 1, 2 oder 3, insbesondere 0 oder 1, darstellt.

Die vorgenannten Reste X erlauben im Rahmen der vorliegenden Erfindung die Bereitstellung bidentater Verbindungen, die, als Liganden in Hydroformylierungen eingesetzt, eine effiziente Prozessführung gestatten.

Ferner ist es vorteilhaft so, dass die Verwendung der Verbindungen als Liganden in Hydroformylierungen mit hohen Umsätzen und vorteilhaften Selektivitäten als Ergebnis einer effektiven Koordination an das Katalysatormetall einhergehen kann.

Hierbei ist es erfindungsgemäß weiter bevorzugt, wenn X ausgewählt ist aus einem der folgenden Reste:
- X ist ein aliphatischer Rest gemäß allgemeiner Formel (III a) 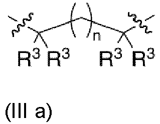
   wobei R³, gleich oder verschieden und/oder unabhängig voneinander, jeweils ausgewählt ist aus einem der folgenden Reste: H und C₁-C₅-Alkyl, insbesondere H, Methyl und tert-Butyl,
   wobei n eine ganze Zahl 1 oder 2 darstellt;
- X ist ein aliphatischer Rest gemäß allgemeiner Formel (III b) 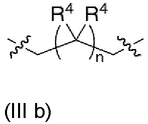
   wobei R³ jeweils = H ist,
   wobei R⁴, gleich oder verschieden und/oder unabhängig voneinander, jeweils ausgewählt ist aus einem der folgenden Reste: H und C₁-C₅-Alkyl, insbesondere H, Methyl und *tert*-Butyl, und wobei n eine ganze Zahl 1 oder 2 darstellt,
- X ist ein aromatischer Rest gemäß allgemeiner Formel (IV a) 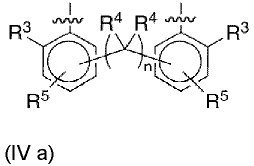
   wobei R³, gleich oder verschieden und/oder unabhängig voneinander, jeweils ausgewählt ist aus einem der folgenden Reste: H und C₁-C₅-Alkyl, insbesondere H, Methyl und *tert*-Butyl,
   wobei R⁴, gleich oder verschieden und/oder unabhängig voneinander, jeweils ausgewählt ist aus einem der folgenden Reste: H und C₁-C₅-Alkyl, insbesondere H, Methyl und *tert*-Butyl, oder wobei zwei Reste R⁴ einen gemeinsam Ring ausbilden, insbesondere einen gemeinsamen aliphatischen C₅- oder C₆-Ring,
   wobei R⁵, gleich oder verschieden und/oder unabhängig voneinander, jeweils ausgewählt ist aus einem der folgenden Reste: H, C₁-C₅-Alkyl und C₁-C₅-Alkoxy, insbesondere H, Methyl, Methoxy und *tert*-Butyl, und
   wobei n eine ganze Zahl 0, 1, 2 oder 3, insbesondere 0 oder 1, darstellt.

Besonders gute Ergebnisse werden im Rahmen der vorliegenden Erfindung erzielt, wenn X ausgewählt ist aus einem der folgenden Reste:

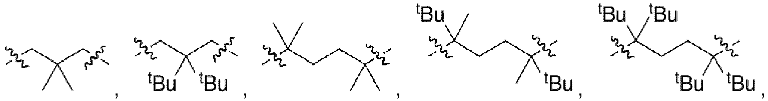

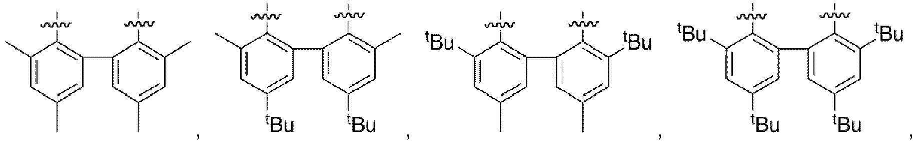

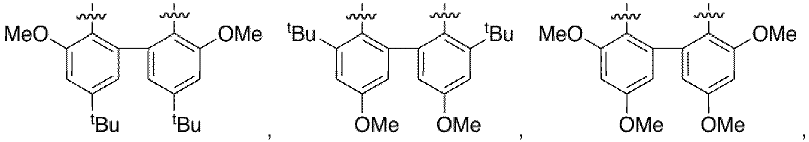

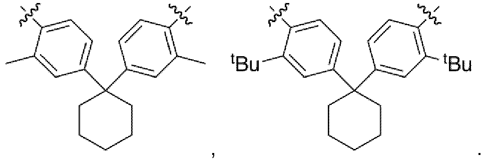

Dabei hat es sich im Rahmen der vorliegenden Erfindung speziell bewährt, wenn X ausgewählt ist aus einem der folgenden Reste:

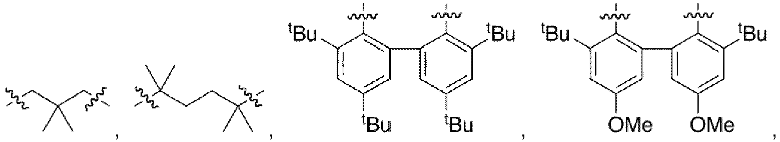

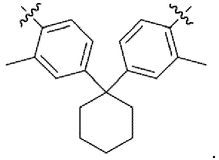

Ebenfalls hat es sich im Rahmen der vorliegenden Erfindung besonders bewährt, wenn X ausgewählt ist aus einem der folgenden Reste:

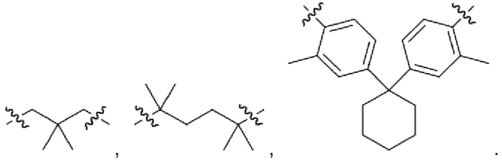

Die erfindungsgemäße Verbindungen zeichnen sich also vorteilhaft dadurch aus, das überwiegend sterisch anspruchsvolle Reste bzw. Strukuren verwendet werden. Ebenfalls vorteilhaft ist eine gewisse Flexibiltiät des Restes X bzw. eine gewisse Bewegungsfreiheit der Bindungen zwischen C-Atomen innerhalb des Restes X.

Im Rahmen einer besonders bevorzugten Ausführung der vorliegenden Erfindung ist es schließlich vorgesehen, dass die Verbindung gemäß Formel (I) ausgewählt ist aus einer der folgenden Verbindungen, wobei in den nachfolgenden Formeln die Reste R¹ und R², gleich oder verschieden und/oder unabhängig voneinander, jeweils ausgewählt sind aus einem der folgenden Reste: H, Methyl und *tert*-Butyl, bevorzugt Methyl:

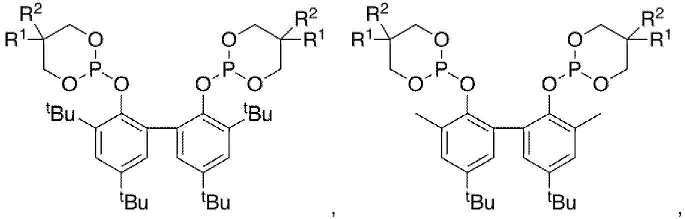

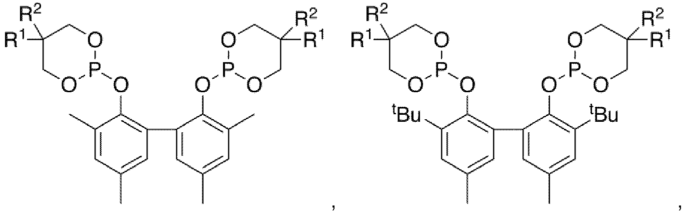

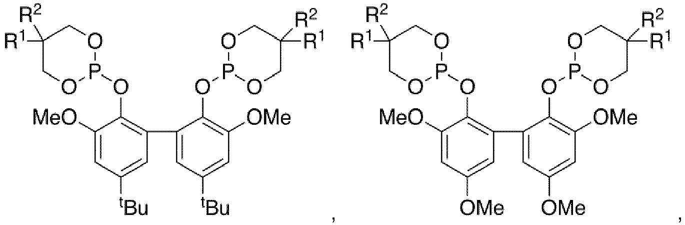

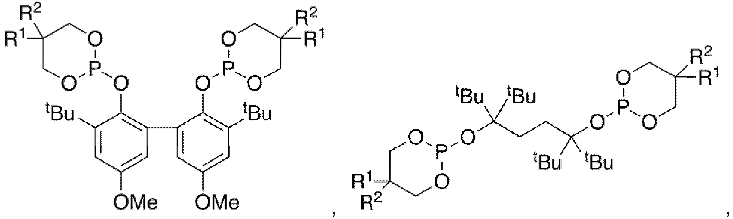

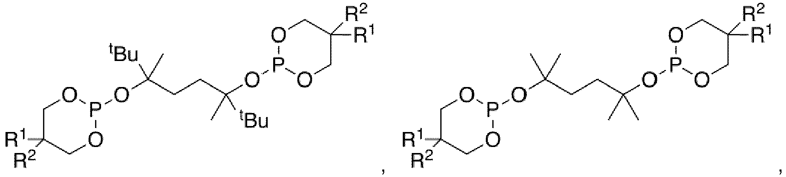

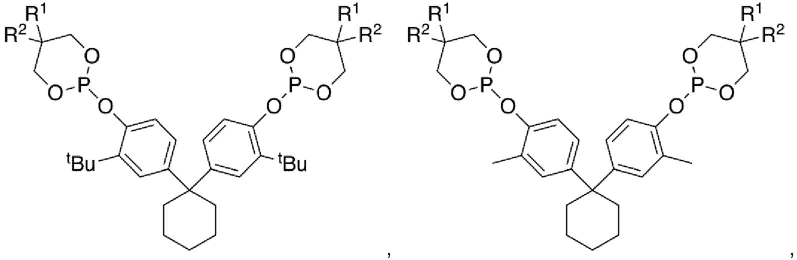

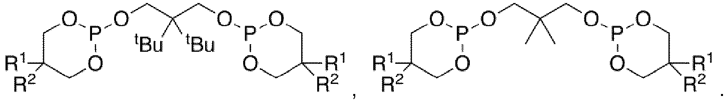

In einer noch weiter bevorzugten Ausführung der vorliegenden Erfindung ist die Verbindung gemäß Formel (I) ausgewählt aus einer der folgenden Verbindungen:

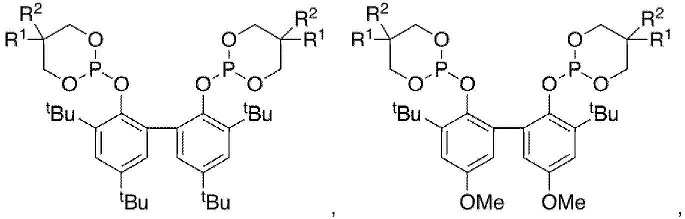

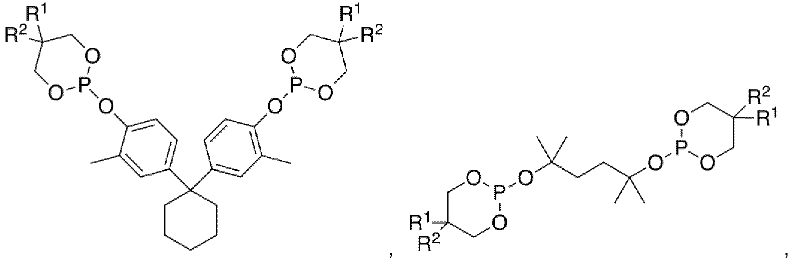

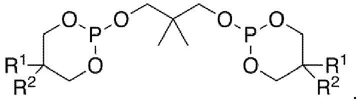

In einer wiederum noch weiter bevorzugten Ausführung der vorliegenden Erfindung ist die Verbindung gemäß Formel (I) ausgewählt aus einer der folgenden Verbindungen:

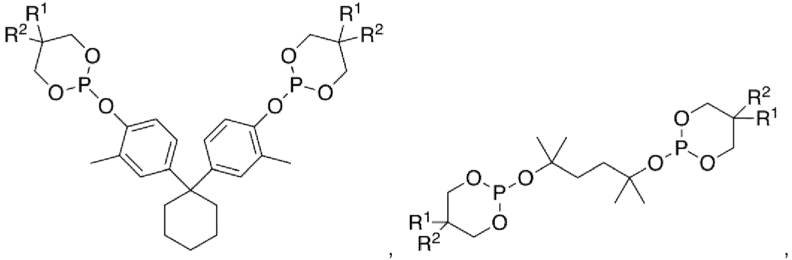

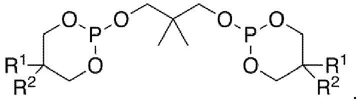

Auf Basis der zuvor beschriebenen erfindungsgemäßen Verbindungen mit den vorgenannten speziellen Resten sowie bevorzugten Strukturmerkmalen werden die mit der vorliegenden Erfindung assoziierten, zuvor bereits genannten Vorteile und technischen Besonderheiten in besonderem Maße realisiert.

Abschließend wird gemäß einer besonders bevorzugten Ausführungsform der Erfindung auf Folgendes hingewiesen: Bei allen vorgenannten Verbindungen nach der vorliegenden Erfindung, insbesondere bei allen vorgenannten Verbindungen der Formeln (I), (IV) und (IV a), ist es bevorzugt, wenn mindestens eine, insbesondere beide, der nachfolgend genannten Verbindungen ausgenommen sind (sog. Disclaimer):

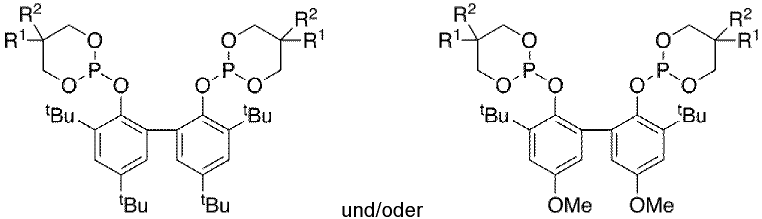

Weiterer Gegenstand der vorliegenden Erfindung - gemäß einem **zweiten** Aspekt der vorliegenden Erfindung - ist ein Verfahren zur Herstellung einer Verbindung gemäß Formel (I), wobei eine reaktive Phosphor(III)spezies, insbesondere ein Phosphor(III)halogenid, umgesetzt wird mit einem Hydroxylgruppen-haltigen Reagenz, so dass eine Verbindung gemäß Formel (I) resultiert.

Vorzugsweise ist es dabei vorgesehen, dass die reaktive Phosphor(III)spezies, insbesondere das Phosphor(III)halogenid, die Verbindung gemäß allgemeiner Formel (V) 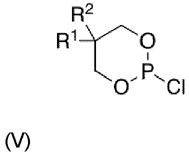
mit Resten R¹ und R², wie zuvor definiert, ist, und/oder
dass das Hydroxylgruppen-haltige Reagenz eine Verbindung gemäß allgemeiner Formel (VI) 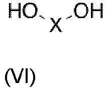
mit X, wie zuvor definiert, ist.

Gute Ergebnisse werden dabei erhalten, wenn das Verfahren bei einer Temperatur im Bereich von -25 °C bis 50 °C und/oder in einem, bevorzugt organischen, insbesondere aprotischen, Lösemittel durchgeführt wird.

Weiter hat es sich bewährt, wenn das Verfahren in Gegenwart einer Base, insbesondere einer metallorganischen Verbindung wie n-Butyllithium, durchgeführt wird.

Für weitergehende Einzelheiten zu diesem Erfindungsaspekt kann auf die obigen Ausführungen und Vorteile zu den Verbindungen nach der vorliegenden Erfindung verwiesen werden, welche in Bezug auf das erfindungsgemäße Verfahren entsprechend gelten.

Wiederum weiterer Gegenstand der vorliegenden Erfindung - gemäß einem **dritten** Aspekt der vorliegenden Erfindung - ist eine katalytisch aktive Verbindung, wobei die katalytisch aktive Verbindung mindestens ein Übergangsmetall, insbesondere ausgewählt aus der 8. bis 11. Gruppe des Periodensystems, vorzugsweise ausgewählt aus der 9. Gruppe des Periodensystems, bevorzugt Rhodium, zusammen mit mindestens einer zuvor beschriebenen Verbindung als Ligand umfasst.

Für weitergehende Einzelheiten zu diesem Erfindungsaspekt kann auf die obigen Ausführungen und Vorteile zu den Verbindungen nach der vorliegenden Erfindung verwiesen werden, welche in Bezug auf das erfindungsgemäße Verfahren entsprechend gelten.

Ebenfalls weitere Gegenstände der vorliegenden Erfindung - gemäß einem **vierten** Aspekt bzw. einem **fünften** Aspekt der vorliegenden Erfindung - sind die Verwendung einer Verbindung gemäß Formel (I), wie zuvor beschrieben, oder einer katalytisch aktiven Verbindung, wie zuvor beschrieben, in einem Hydroformylierungsverfahren, insbesondere zur Umsetzung ethylenisch ungesättigter Verbindungen zu den entsprechenden Aldehyden, bzw. ein Verfahren zur Hydroformylierung, wobei mindestens eine ethylenisch ungesättigte Verbindung in Gegenwart einer Verbindung gemäß Formel (I), wie zuvor beschrieben, oder einer katalytisch aktiven Verbindung, wie zuvor beschrieben, einer Hydroformylierung unterzogen wird, insbesondere zu dem entsprechenden Aldehyd umgesetzt wird.

Hierbei hat es sich sowohl für die Verwendung als auch das Verfahren nach der vorliegenden Erfindung bewährt, wenn das Hydroformylierungsverfahren die Verfahrensschritte:
a) Vorlegen einer ethylenisch ungesättigten Verbindung;
b) Zugabe einer Verbindung gemäß Formel (I), wie zuvor beschrieben, und einer Substanz, welche mindestens ein Übergangsmetall, insbesondere ausgewählt aus der 8. bis 11. Gruppe des Periodensystems, vorzugsweise ausgewählt aus der 9. Gruppe des Periodensystems, bevorzugt Rhodium, umfasst, oder einer katalytisch aktiven Verbindung, wie zuvor beschrieben;
c) Zuführen von H₂ und CO,
d) Erwärmen des Reaktionsgemisches aus a) bis c), wobei das Olefin zu einem Aldehyd umgesetzt wird,
umfasst.

Weiter werden im Rahmen der Verwendung sowie des Verfahrens nach der vorliegenden Erfindung gute Ergebnisse erhalten, wenn die ethylenisch ungesättigte Verbindung in Verfahrensschritt a) ausgewählt ist aus: Ethen, Propen, 1-Buten, *cis-* und/oder *trans*-2-Buten, *iso*-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, Di-n-buten, oder Mischungen davon.

Auch hat es sich für die genannten Erfindungsaspekte bewährt, wenn die Substanz, welche mindestens ein Übergangsmetall, insbesondere ausgewählt aus der 8. bis 11. Gruppe des Periodensystems, vorzugsweise ausgewählt aus der 9. Gruppe des Periodensystems, bevorzugt Rhodium, umfasst, ausgewählt ist aus: Rh(acac)(CO)₂ und/oder [(acac)Rh(COD)], wobei acac = Acetylacetonat-Anion und COD = 1,5-Cyclooctadien sind, und/oder Rh₄CO₁₂, oder aber
wenn die katalytisch aktive Verbindung neben der mindestens eine Verbindung gemäß Formel (I) mindestens einen weiteren Liganden ausgewählt aus (acac), (CO) und/oder (COD) ), wobei acac = Acetylacetonat-Anion und COD = 1,5-Cyclooctadien sind, umfasst.

Vorzugsweise ist außerdem vorgesehen, dass das Zuführen von CO in Verfahrensschritt c) mit einem Druck in dem Bereich von 1 bis 6 MPa (10 bis 60 bar) erfolgt.

Ebenfalls ist es bevorzugt, wenn das Erwärmen des Reaktionsgemisches in Verfahrensschritt d) auf eine Temperatur in dem Bereich von 80 °C bis 160 °C erfolgt.

Für weitergehende Einzelheiten zu diesem Erfindungsaspekt kann auf die obigen Ausführungen und Vorteile zu den Verbindungen nach der vorliegenden Erfindung verwiesen werden, welche in Bezug auf das erfindungsgemäße Verfahren entsprechend gelten.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, jedoch ohne die vorliegende Erfindung hierauf zu beschränken.

### Ausführungsbeispiele:

Nachfolgend ist die Herstellung erfindungsgemäßer Verbindungen sowie deren Verwendung in einem Verfahren zur Hydroformylierung beschrieben.

### 1. Herstellung von 2,2'-((3,3'-Di-tert.-butyl-5,5'-dimethoxy-[1,1'-biphenyl]-2,2'-diyl)bis(oxy))bis(5,5-dimethyl-1,3,2-dioxaphosphinan) (Verbindung 1)

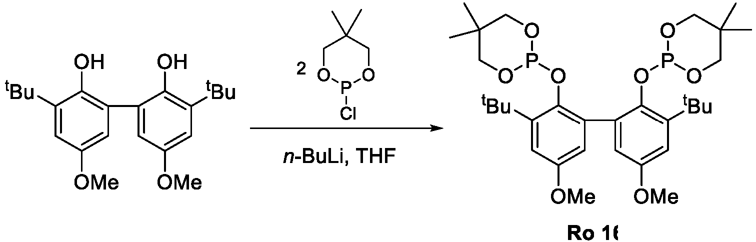

Zu einer Lösung von 3,3'-Di-*tert*-butyl-5,5'-dimethoxy-[1,1'-biphenyl]-2,2'-diol (251 mg, 0.70 mmol) in THF (2 ml) gibt man bei -20 °C eine Lösung von *n*-Butyllithium in Hexan (0.53 M, 2.75 ml, 1.47 mmol) und lässt nach 20 Minuten auf Raumtemperatur erwärmen. Dann versetzt man das Reaktionsgemisch langsam mit einer Lösung des 2-Chloro-5,5-dimethyl-1,3,2-dioxaphosphinans (248 mg, 1.47 mmol) in THF (3 ml). Nach dem Rühren über Nacht wird das Lösemittel im Vakuum entfernt und Rückstand anschließend mit Toluol (6 ml) aufgenommen. Die unlöslichen Bestandteile werden durch Filtration (G4-Fritte) entfernt und das Filtrat erneut eingeengt und der Rückstand getrocknet. Durch Säulenchromatographie (Eluent: Heptan/Dichlormethan 1/2) wird das Produkt isoliert.

Ausbeute: 255 mg weißer Feststoff (58%).

³¹P-NMR (CD₂Cl₂): d +116.6 ppm.

¹H-NMR (CD₂Cl₂): d 6.95 (2H, d, *J* 3.2 Hz, 2xHₐᵣ), 6.62 (2H, d, *J* 3.2 Hz, 2xHₐᵣ), 4.02 (4H, m, 4xHₐ-CH₂O), 3.74 (6H, s, 2xOCH₃), 3.14 (4H, m, 4xH_{b}-CH₂O), 1.45 (18H, s, 2xC(CH₃)₃), 1.13 (6H, s, 2xCH₃), 0.67 (6H, s, 2xCH₃).

¹³C-NMR (CD₂Cl₂): d 154.4 (2xCₐᵣ-OMe), 145.2 (t, *J* 3.8 Hz, 2xCₐᵣ), 143.1 (2xCₐᵣ), 134.2 (t, *J* 3.0 Hz, 2xCₐᵣ), 115.3 (2xCₐᵣH), 114.8 (2xCₐᵣH), 69.9 (d, *J* 2.0 Hz, 4xCH₂O), 55.9 (2xOCH₃), 35.5 (2x^{t}C), 32.6 (t, *J* 4.2 Hz, 2x^{t}C), 30.0 (6xCH₃), 22.9 (2xCH₃), 22.6 (2xCH₃).

HRMS (ESI-TOF) [M+H]⁺: *m*/*z* berechnet: für C₃₂H₄₈O₈P₂ 623.2903, gefunden: 623.2909.

HRMS (ESI-TOF) [M+Na]⁺: *m*/*z* berechnet: für C₃₂H₄₈O₈P₂ 645.2717, gefunden: 645.2734.

| | | | |
|---|---|---|---|
| Elementaranalyse: | berechnet: | C 61.73% H 7.77% | P 9.95% |
| | gefunden: | C 61.85% H 7.62% | P 9.70% |

### 2. Herstellung von 2,2'-((3,3',5,5'-Tetra-tert-butyl-[1,1'-biphenyl]-2,2'-diyl)bis(oxy))bis(5,5-dimethyl-1,3,2-dioxaphosphinan) (Verbindung 2)

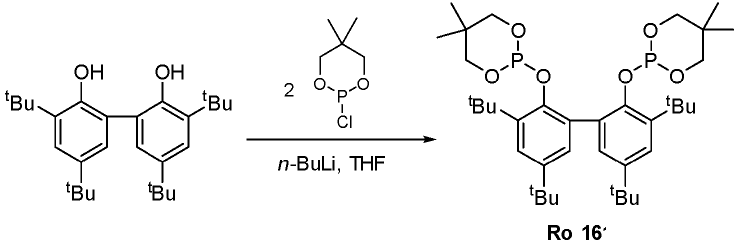

Zu einer Lösung von 3,3',5,5'-Tetra-*tert*-butyl-[1,1'-biphenyl]-2,2'-diol (350 mg, 0.85 mmol) in THF (2 ml) gibt man bei -20 °C eine Lösung von n-Butyllithium in Hexan (0.53 M, 3.36 ml, 1.79 mmol) und lässt nach 20 Minuten auf Raumtemperatur erwärmen. Das Reaktionsgemisch wird langsam mit einer Lösung des 2-Chloro-5,5-dimethyl-1,3,2-dioxaphosphinans (301 mg, 1.79 mmol) in THF (3 ml) versetzt. Nach dem Rühren über Nacht wird das Lösemittel im Vakuum entfernt und Rückstand anschließend mit Toluol (6 ml) aufgenommen. Die unlöslichen Bestandteile werden abfiltriert (G4-Fritte), das Filtrat erneut eingeengt und der feste Rückstand getrocknet. Durch Säulenchromatographie (Eluent: Heptan/Dichlormethan 1/2) wird das Produkt isoliert.

Ausbeute: 445 mg weißer Feststoff (77%).

³¹P-NMR (CD₂Cl₂): d +115.4 ppm.

¹H-NMR (CD₂Cl₂): d 7.41 (2H, d, *J* 2.6 Hz, 2xHₐᵣ), 7.07 (2H, m, 2xHₐᵣ), 3.94 (4H, m, 4xHₐ-CH₂O), 3.05 (4H, m, 4xH_{b}-CH₂O), 1.47 (18H, s, 2xC(CH₃)₃), 1.32 (18H, s, 2xC(CH₃)₃), 1.10 (6H, s, 2xCH₃), 0.64 (6H, s, 2xCH₃).

¹³C-NMR (CD₂Cl₂): d 149.1 (t, *J* 3.5 Hz, 2xCₐᵣ-O), 144.6 (2xCₐᵣ), 140.7 (2xCₐᵣ), 133.4 (t, *J* 2.7 Hz, 2xCₐᵣ), 129.1 (2xCₐᵣH), 124.3 (2xCₐᵣH), 69.7 (d, *J* 3.6 Hz, 4xCH₂O), 35.5 (2x^{t}C), 34.7 (2x^{t}C), 32.5 (t, *J* 2.2 Hz, ^{t}C), 31.6 (6xCH₃), 31.2 (6xCH₃), 22.9 (CH₃), 22.6 (CH₃).

HRMS (ESI-TOF) [M+H]⁺: *m*/*z* berechnet: für C₃₈H₆₀O₆P₂ 675.3943, gefunden: 675.3948.

HRMS (ESI-TOF) [M+Na]⁺: *m*/*z* berechnet: für C₃₈H₆₀O₆P₂ 697.3757, gefunden: 697.3769.

| | | | |
|---|---|---|---|
| Elementaranalyse: | berechnet: | C 67.64% H 8.96% | P 9.18% |
| | gefunden: | C 67.63% H 9.10% | P 9.15% |

### 3. Herstellung von 2,2'-((2,2-Dimethylpropan-1,3-diyl)bis(oxy))bis(5,5-dimethyl-1,3,2-dioxaphosphinan) (Verbindung 3)

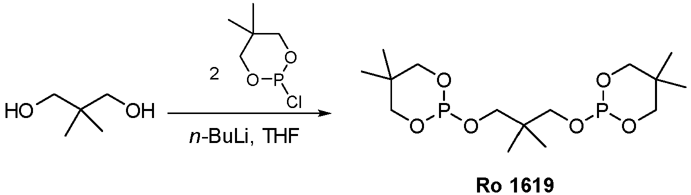

Eine Lösung des 2,2-Dimethylpropan-1,3-diols (141 mg, 1.36 mmol) in THF (2 ml) wird auf -20 °C abgekühlt und langsam mit einer Lösung von *n*-Butyllithium in Hexan (1.6 M, 1.78 ml, 2.85 mmol) versetzt. Nach etwa 20 Minuten wird die trübe Lösung mit dem 2-Chloro-5,5-dimethyl-1,3,2-dioxaphosphinan (481 mg, 2.85 mmol) in THF (5 ml) bei Raumtemperatur versetzt. Nach dem Rühren über Nacht bei Raumtemperatur wird das Lösemittel unter Vakuum entfernt und der Rückstand mit Toluol (7 ml) aufgenommen. Nach der Filtration wird der viskose Rückstand unter Vakuum bei 60 °C getrocknet. Eine weitere Reinigung erfolgte nicht, da das Produkt mit einer Reinheit von 96% (NMR) vorlag.

Ausbeute: 470 mg weißer Feststoff (94%).

³¹P-NMR (CD₂Cl₂): d +122.0 ppm.

¹H-NMR (CD₂Cl₂): d 4.13 (4H, m, 4xHₐ-CH₂O), 3.57 (4H, m, 2xCH₂O), 3.28 (4H, m, 4xH_{b}-CH₂O),1.22 (6H, s, 2xCH₃), 0.97 (6H, s, 2xCH₃), 0.72 (6H, s, 2xCH₃).

¹³C-NMR (CD₂Cl₂): d 69.2 (4xCH₂O), 68.0 (d, *J* 17.0 Hz, 2xCH₂O), 37.3 (t, *J* 6.3 Hz,2x^{t}C), 33.1 (d, *J* 5.3 Hz, ^{t}C), 22.9 (2xCH₃), 22.6 (2xCH₃), 21.7 (2xCH₃).

HRMS (ESI-TOF) [M+Na]⁺: *m*/*z* berechnet: für C₁₅H₃₀O₆P₂ 391.1410, gefunden: 391.1412.

| | | | |
|---|---|---|---|
| Elementaranalyse: | berechnet: | C 48.91% H 8.21 % | P 16.82% |
| | gefunden: | C 48.71% H 8.48% | P 17.05% |

### 4. Herstellung von 2,2'-((Cyclohexan-1,1-diylbis(2-methyl-4,1-phenylene))bis(oxy))bis(5,5-dimethyl-1,3,2-dioxaphosphinan) (Verbindung 4)

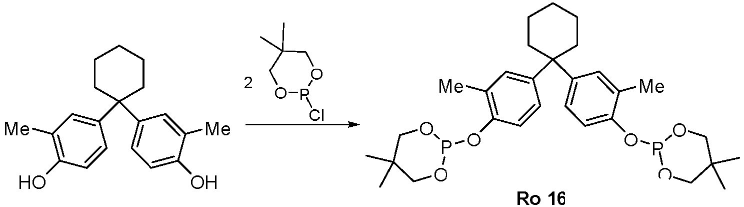

Eine Lösung von 4,4'-(Cyclohexan-1,1-diyl)bis(2-methylphenol) (389 mg, 1.31 mmol) und Triethylamin (398 mg, 3.94 mmol) in THF (3 ml) wird bei 0 °C vorgelegt und anschließend langsam mit einer Lösung des 2-Chloro-5,5-dimethyl-1,3,2-dioxaphosphinans (464 mg, 2.75 mmol) in THF (3 ml) versetzt. Man lässt die Reaktionsmischung auf Raumtemperatur erwärmen und rührt über Nacht weiter. Nach der Filtration wird das Lösemittel unter Vakuum entfernt und der feste Rückstand unter Vakuum getrocknet. Das Produkt lag in einer Reinheit von >98% (³¹P-NMR) vor und wurde nicht weiter gereinigt.

Ausbeute: 550 mg weißer Feststoff (75%).

³¹P-NMR (CD₂Cl₂): d +115.6 ppm.

¹H-NMR (CD₂Cl₂): d 7.15 (2H, m, 2xHₐᵣ), 7.07 (2H, m, 2xHₐᵣ), 6.98 (2H, m, 2xHₐᵣ), 4.35 (4H, m, 4xHₐ-CH₂O), 3.45 (4H, m, 4xH_{b}-CH₂O), 2.27-2.21 (4H, m, 2xCH₂), 2.27 (6H, s, 2xCH₃), 1.63-1.44 (6H, m, 3xCH₂), 1.31 (6H, s, 2xCH₃), 0.79 (6H, s, 2xCH₃).

¹³C-NMR (CD₂Cl₂): d 149.1 (d, *J* 6.6 Hz, 2xCₐᵣ-O), 144.0 (2xCₐᵣ), 130.1 (2xCₐᵣH), 129.3 (d, *J* 2.3 Hz, 2xCₐᵣ), 125.8 (2xCₐᵣH), 118.9 (d, *J* 12.9 Hz, 2xCₐᵣH), 69.7 (d, *J* 2.4 Hz, 4xCH₂O), 45.4 (^{t}C), 37.5 (2xCH₂), 33.1 (d, *J* 5.1 Hz, 2x^{t}C), 26.8 (CH₂), 23.4 (2xCH₂), 22.9 (2xCH₃), 22.6 (2xCH₃), 17.3 (2xCH₃).

HRMS (ESI-TOF) [M+H]⁺: *m*/*z* berechnet: für C₃₀H₄₂O₆P₂ 561.2535, gefunden: 561.2539.

HRMS (ESI-TOF) [M+Na]⁺: *m*/*z* berechnet: für C₃₀H₄₂O₆P₂ 583.2349, gefunden: 583.2360.

| | | | |
|---|---|---|---|
| Elementaranalyse: | berechnet: | C 64.27% H 7.55% | P 11.05% |
| | gefunden: | C 64.28% H 7.52% | P 11.17 |

### 5. Herstellung von 2,2'-((2,5-Dimethylhexan-2,5-diyl)bis(oxy))bis(5,5-dimethyl-1,3,2-dioxaphosphinan) (Verbindung 5)

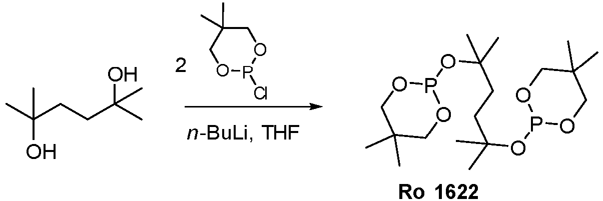

Eine Lösung des 2,5-Dimethylhexane-2,5-diols (221 mg, 1.51 mmol) in THF (2 ml) wird auf -20 °C abgekühlt und langsam mit einer Lösung von *n*-Butyllithium in Hexan (1.6 M, 1.98 ml, 3.17 mmol) versetzt. Nach etwa 20 Minuten wird die Reaktionsmischung mit dem 2-Chloro-5,5-dimethyl-1,3,2-dioxaphosphinan (534 mg, 3.17 mmol) in THF (4 ml) bei Raumtemperatur versetzt. Nach dem Rühren über Nacht bei Raumtemperatur wird das Lösemittel unter Vakuum entfernt und der Rückstand mit Toluol (7 ml) aufgenommen. Nach der Filtration wird der viskose Rückstand unter Vakuum bei 60 °C getrocknet. Das Produkt lag in einer Reinheit von 98% (³¹P- NMR) vor und wurde nicht weiter gereinigt.

Ausbeute: 595 mg weißer Feststoff (96%).

³¹P-NMR (CD₂Cl₂): d +117.6 ppm.

¹H-NMR (CD₂Cl₂): d 4.18 (4H, m, 4xHₐ-CH₂O), 3.23 (4H, m, 4xH_{b}-CH₂O), 1.76 (4H, s, 2xCH₂), 1.40 (12H, s, 4xCH₃), 1.21 (6H, s, 2xCH₃), 0.71 (6H, s, 2xCH₃).

¹³C-NMR (CD₂Cl₂): d 77.9 (d, *J* 6.8 Hz, 2x^{t}CO), 68.6 (4xCH₂O), 38.4 (d, *J* 5.2 Hz, 2xCH₂), 33.1 (d, 4.3 Hz, 2x^{t}C), 33.0 (2x^{t}C), 29.1 (d, *J* 10.1 Hz, 4xCH₃), 23.0 (2xCH₃), 22.7 (2xCH₃).

HRMS (ESI-TOF) [M+Na]⁺: *m*/*z* berechnet: für C₁₈H₃₆O₆P₂ 433.1884, gefunden: 433.1887.

| | | | |
|---|---|---|---|
| Elementaranalyse: | berechnet: | C 52.68% H 8.84% | P 15.09% |
| | gefunden: | C 52.58% H 8.53% | P 15.20 |

### 6. Durchführung von Katalyse-Versuchen mit den Liganden gemäß Verbindungen 1 bis 4

### a) Durchführung:

Die Hydroformylierung wurde in einem mit Druckkonstanthaltung, Gasflussmessung,

Begasungsrührer und Druckpipette ausgestatteten 200 ml-Autoklaven der Fa. Premex Reactor AG, Lengau, Schweiz, durchgeführt.

Zur Minimierung eines Einflusses von Feuchtigkeit und Sauerstoff wurde das als Solvens benutzte Toluol in einem Pure Solv. MD-7 System gereinigt und unter Argon aufbewahrt.

Cis/trans-2-Penten (Aldrich) wurde über Natrium am Rückfluss erhitzt und unter Argon destilliert. Im Autoklav wurden unter Argonatmosphäre Lösungen der Katalysatorvorstufe und des Liganden jeweils in Toluol gemischt.

Als Katalysatorvorstufe kam [(acac)Rh(COD)] (Umicore, acac = Acetylacetonat-Anion; COD = 1,5-Cyclooctadien), zum Einsatz.

Der Autoklav wurde bei einem Gesamtgasdruck (Synthesegas: Linde; H₂ (99,999%) : CO (99,997%) = 1:1) von 12 bar für den Enddruck von 20 bar unter Rühren (1500 U/min) aufgeheizt. Nach Erreichen der Reaktionstemperatur wurde das in der Druckpipette befindliche Olefin mit einem Überdruck von ca. 3 bar in den Autoklaven gepresst. Die Reaktion wurde bei konstantem Druck (Nachdruckregler der Fa. Bronkhorst, NL) über 4 h geführt. Der Autoklav wurde nach Ablauf der Reaktionszeit auf Zimmertemperatur abgekühlt, unter Rühren entspannt und mit Argon gespült.

Jeweils 1 ml der Reaktionsmischung wurden entnommen, mit 10 ml Pentan verdünnt und gaschromatographisch analysiert: HP 5890 Series II plus, PONA, 50 m x 0,2 mm x 0,5 µm.

### b) Ergebnisse in der Hydroformylierung von 2-Penten:

Für die Verbindungen 1 bis 4 wurden die folgenden Umsätze und n-Selektivitäten bestimmt, wobei n-Selektivität die Selektivität für die Bildung linearer Aldehyde angibt und aus dem Verhältnis n-Aldehyd : i-Aldehyd ermittelt werden kann.

| **Verbindung** | **Umsatz (%)** | ***n*-Selektivität (%)** |
|---|---|---|
| 1 | 94 | 34.2 |
| 2 | 98 | 20.1 |
| 3 | 97 | 8.1 |
| 4 | 94 | 19.9 |

Standardbedingungen: Substrat: 2-Penten, T: 120°C, *t*: 4 h, 20 bar CO/H₂ (1:1), Solvens Toluol, 100 ppm Rh, [Olefin]= 2,41 M, Rh/Ligand 1/2;

## Patentansprüche

1. Verbindung gemäß allgemeiner Formel (I) 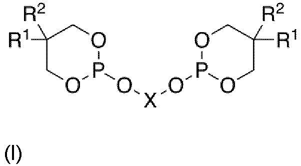
wobei R¹ und R², gleich oder verschieden und/oder unabhängig voneinander, jeweils ausgewählt sind aus einem der folgenden Reste: H und C₁-C₅-Alkyl, insbesondere H, Methyl und *tert*-Butyl, und
wobei X ein zweibindiger organischer Rest, insbesondere ein gesättigter oder ungesättigter, aliphatischer oder aromatischer, zweibindiger organischer Rest, ist;
insbesondere wobei die Verbindung symmetrisch, insbesondere punktsymmetrisch oder spiegelsymmetrisch, ausgebildet ist.

2. Verbindung nach Anspruch 1,
wobei X ausgewählt ist aus einem der folgenden Reste:
- X ist ein aliphatischer Rest gemäß allgemeiner Formel (III) 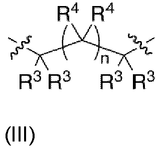
wobei R³, gleich oder verschieden und/oder unabhängig voneinander, jeweils ausgewählt ist aus einem der folgenden Reste: H und C₁-C₅-Alkyl, insbesondere H, Methyl und *tert*-Butyl, wobei R⁴, gleich oder verschieden und/oder unabhängig voneinander, jeweils ausgewählt ist aus einem der folgenden Reste: H und C₁-C₅-Alkyl, insbesondere H, Methyl und *tert*-Butyl, oder wobei zwei Reste R⁴ einen gemeinsamen Ring ausbilden, insbesondere einen gemeinsamen aliphatischen C₅- oder C₆-Ring, und
wobei n eine ganze Zahl 0, 1, 2 oder 3, insbesondere 1 oder 2, darstellt;
- X ist ein Rest der allgemeinen Formel (IV) 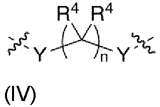
wobei Y ein gegebenenfalls substituierter aromatischer Rest, insbesondere ein gegebenenfalls substituierter Phenylenrest, vorzugsweise ein gegebenenfalls alkylsubstituierter Phenylenrest, bevorzugt ein gegebenenfalls C₁-C₅-alkylsubstituierter und/oder C₁-C₅-alkoxylsubstituierter Phenylenrest, ist,
wobei R⁴, gleich oder verschieden und/oder unabhängig voneinander, jeweils ausgewählt ist aus einem der folgenden Reste: H und C₁-C₅-Alkyl, insbesondere H, Methyl und *tert*-Butyl, oder wobei zwei Reste R⁴ einen gemeinsamen Ring ausbilden, insbesondere einen gemeinsamen aliphatischen C₅- oder C₆-Ring, und
wobei n eine ganze Zahl 0, 1, 2 oder 3, insbesondere 0 oder 1, darstellt.

3. Verbindung nach Anspruch 1 oder 2,
wobei X ausgewählt ist aus einem der folgenden Reste:
- X ist ein aliphatischer Rest gemäß allgemeiner Formel (III a) 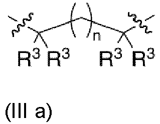 wobei R³, gleich oder verschieden und/oder unabhängig voneinander, jeweils ausgewählt ist aus einem der folgenden Reste: H und C₁-C₅-Alkyl, insbesondere H, Methyl und *tert*-Butyl, wobei n eine ganze Zahl 1 oder 2 darstellt;
- X ist ein aliphatischer Rest gemäß allgemeiner Formel (III b) 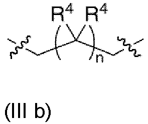
wobei R³ jeweils = H ist,
wobei R⁴, gleich oder verschieden und/oder unabhängig voneinander, jeweils ausgewählt ist aus einem der folgenden Reste: H und C₁-C₅-Alkyl, insbesondere H, Methyl und *tert*-Butyl, und wobei n eine ganze Zahl 1 oder 2 darstellt,
- X ist ein aromatischer Rest gemäß allgemeiner Formel (IV a) 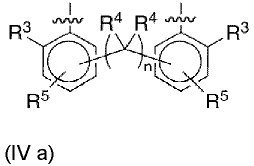
wobei R³, gleich oder verschieden und/oder unabhängig voneinander, jeweils ausgewählt ist aus einem der folgenden Reste: H und C₁-C₅-Alkyl, insbesondere H, Methyl und *tert*-Butyl, wobei R⁴, gleich oder verschieden und/oder unabhängig voneinander, jeweils ausgewählt ist aus einem der folgenden Reste: H und C₁-C₅-Alkyl, insbesondere H, Methyl und *tert*-Butyl, oder wobei zwei Reste R⁴ einen gemeinsam Ring ausbilden, insbesondere einen gemeinsamen aliphatischen C₅- oder C₆-Ring,
wobei R⁵, gleich oder verschieden und/oder unabhängig voneinander, jeweils ausgewählt ist aus einem der folgenden Reste: H, C₁-C₅-Alkyl und C₁-C₅-Alkoxy, insbesondere H, Methyl, Methoxy und *tert*-Butyl, und
wobei n eine ganze Zahl 0, 1, 2 oder 3, insbesondere 0 oder 1, darstellt.

4. Verbindung nach einem der vorangehenden Ansprüche,
wobei X ausgewählt ist aus einem der folgenden Reste: 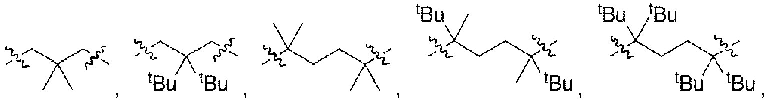 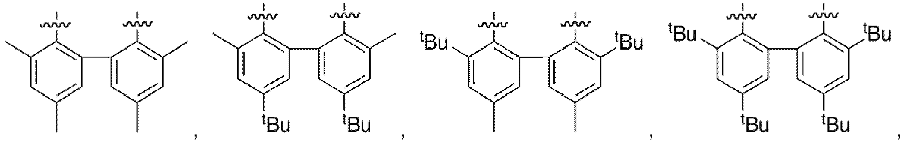 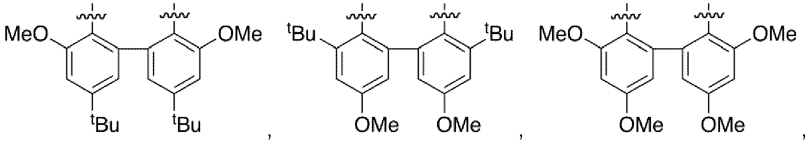 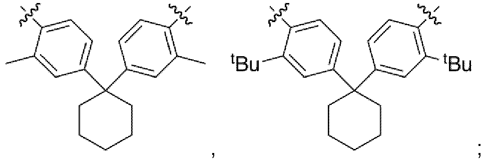
insbesondere wobei X bevorzugt ausgewählt ist aus einem der folgenden Reste: 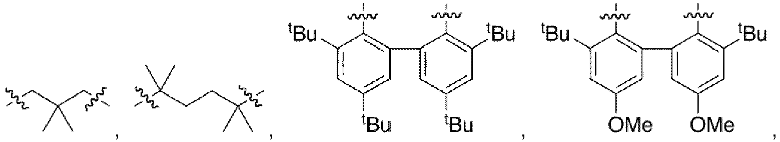 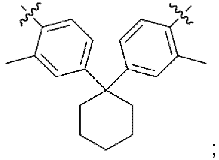
vorzugsweise wobei X bevorzugt ausgewählt ist aus einem der folgenden Reste:

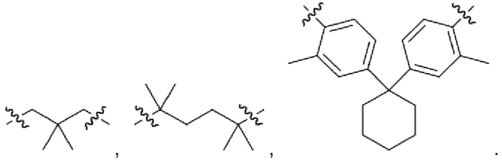

5. Verbindung nach einem der vorangehenden Ansprüche,
wobei die Verbindung gemäß Formel (I) ausgewählt ist aus einer der folgenden Verbindungen, wobei in den nachfolgenden Formeln die Reste R¹ und R², gleich oder verschieden und/oder unabhängig voneinander, jeweils ausgewählt sind aus einem der folgenden Reste: H, Methyl und *tert*-Butyl, bevorzugt Methyl: 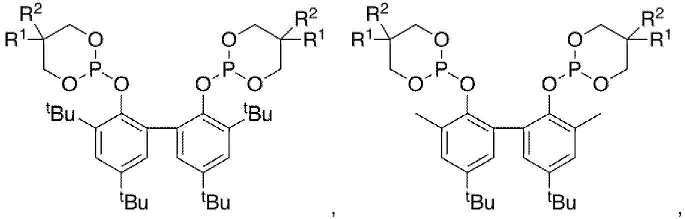 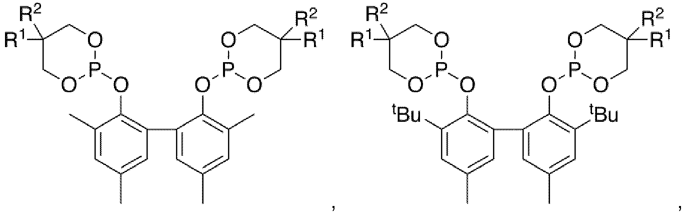 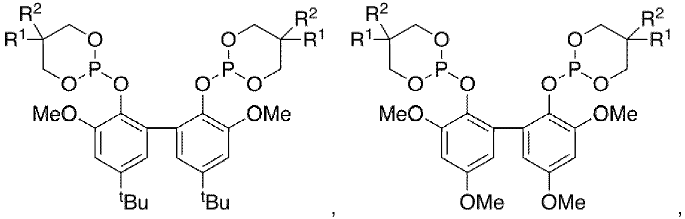 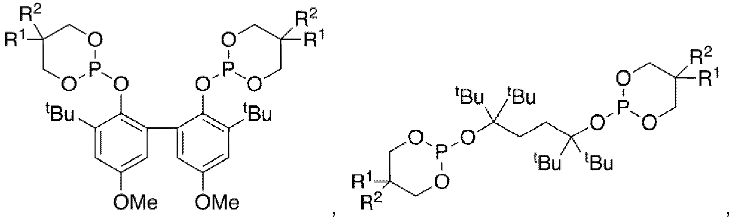 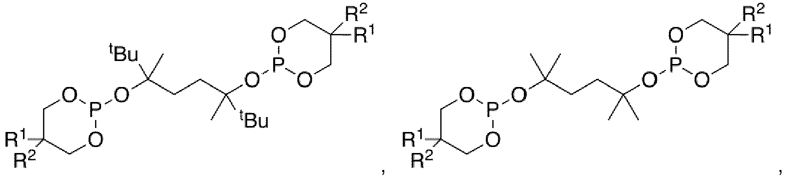 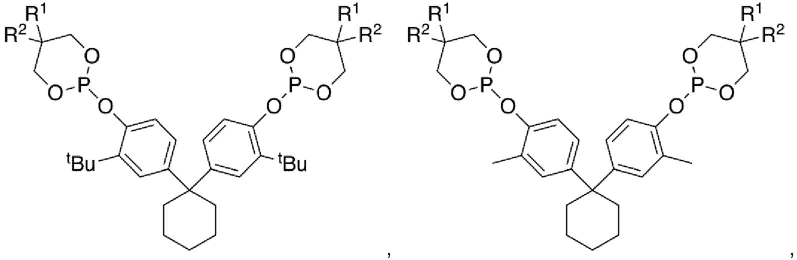 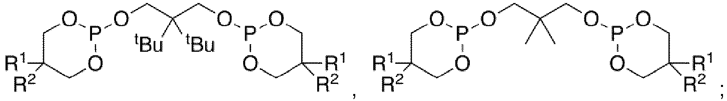
insbesondere wobei die Verbindung gemäß Formel (I) ausgewählt ist aus einer der folgenden Verbindungen: 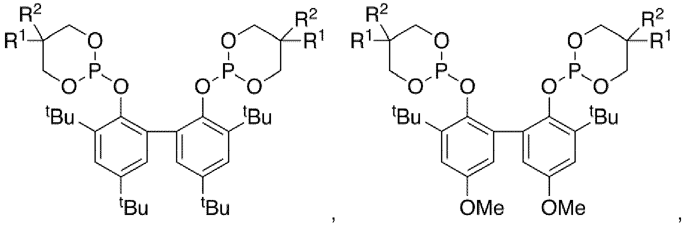 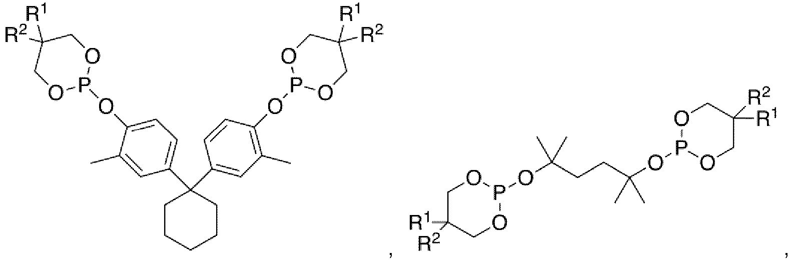 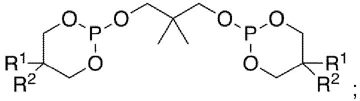
vorzugsweise wobei die Verbindung gemäß Formel (I) ausgewählt ist aus einer der folgenden Verbindungen:

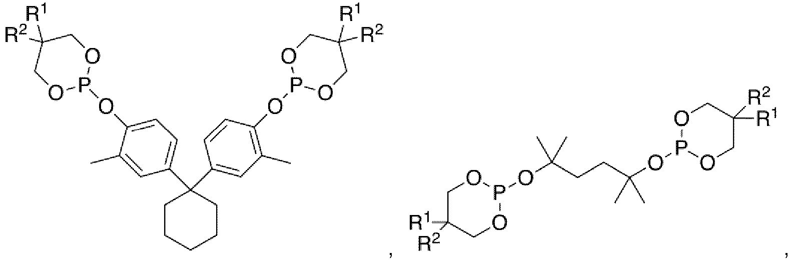

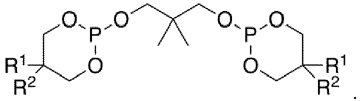

6. Verfahren zur Herstellung einer Verbindung gemäß Formel (I),
wobei eine reaktive Phosphor(III)spezies, insbesondere ein Phosphor(III)halogenid, umgesetzt wird mit einem Hydroxylgruppen-haltigen Reagenz, so dass eine Verbindung gemäß Formel (I) resultiert.

7. Verfahren nach Anspruch 6,
wobei die reaktive Phosphor(III)spezies, insbesondere ein Phosphor(III)halogenid, die Verbindung gemäß allgemeiner Formel (V) 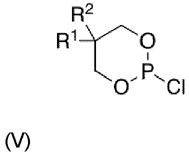
mit Resten R¹ und R², wie zuvor definiert, ist, und/oder
wobei das Hydroxylgruppen-haltige Reagenz eine Verbindung gemäß allgemeiner Formel (VI) 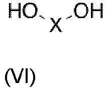
mit X, wie zuvor definiert, ist.

8. Katalytisch aktive Verbindung,
wobei die katalytisch aktive Verbindung mindestens ein Übergangsmetall, insbesondere ausgewählt aus der 8. bis 11. Gruppe des Periodensystems, vorzugsweise ausgewählt aus der 9. Gruppe des Periodensystems, bevorzugt Rhodium, zusammen mit mindestens einer Verbindung gemäß einer der Ansprüche 1 bis 5 als Ligand umfasst.

9. Verwendung einer Verbindung nach einem der Ansprüche 1 bis 5 oder einer katalytisch aktiven Verbindung nach Anspruch 8 in einem Hydroformylierungsverfahren, insbesondere zur Umsetzung ethylenisch ungesättigter Verbindungen zu den entsprechenden Aldehyden.

10. Verfahren zur Hydroformylierung,
wobei mindestens eine ethylenisch ungesättigte Verbindung in Gegenwart einer Verbindung nach einem der Ansprüche 1 bis 5 oder einer katalytisch aktiven Verbindung nach Anspruch 8 einer Hydroformylierung unterzogen wird, insbesondere zu dem entsprechenden Aldehyd umgesetzt wird.

11. Verfahren nach Anspruch 10,
wobei das Hydroformylierungsverfahren die Verfahrensschritte
a) Vorlegen einer ethylenisch ungesättigten Verbindung;
b) Zugabe einer Verbindung gemäß einem der Ansprüche 1 bis 5 und einer Substanz, welche mindestens ein Übergangsmetall, insbesondere ausgewählt aus der 8. bis 11. Gruppe des Periodensystems, vorzugsweise ausgewählt aus der 9. Gruppe des Periodensystems, bevorzugt Rhodium, umfasst, oder einer katalytisch aktiven Verbindung nach Anspruch 8;
c) Zuführen von H₂ und CO,
d) Erwärmen des Reaktionsgemisches aus a) bis c), wobei das Olefin zu einem Aldehyd umgesetzt wird,
umfasst.

12. Verfahren nach einem der Ansprüche 10 oder 11,
wobei die ethylenisch ungesättigte Verbindung in Verfahrensschritt a) ausgewählt ist aus: Ethen, Propen, 1-Buten, *cis-* und/oder *trans*-2-Buten, *iso*-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, Di-n-buten oder Mischungen davon.

13. Verfahren nach einem der Ansprüche 10 bis 12,
wobei die Substanz, welche mindestens ein Übergangsmetall, insbesondere ausgewählt aus der 8. bis 11. Gruppe des Periodensystems, vorzugsweise ausgewählt aus der 9. Gruppe des Periodensystems, bevorzugt Rhodium, umfasst, ausgewählt ist aus: Rh(acac)(CO)₂ und/oder [(acac)Rh(COD)], wobei acac = Acetylacetonat-Anion und COD = 1,5-Cyclooctadien sind, und/oder Rh₄CO₁₂; oder
wobei die katalytisch aktive Verbindung neben der mindestens einen Verbindung gemäß Formel (I) mindestens einen weiteren Liganden, ausgewählt aus (acac), (CO) und/oder (COD), wobei acac = Acetylacetonat-Anion und COD = 1,5-Cyclooctadien sind, umfasst.

14. Verfahren nach einem der Ansprüche 10 bis 13
wobei das Zuführen von CO in Verfahrensschritt c) mit einem Druck in dem Bereich von 1 bis 6 MPa (10 bis 60 bar) erfolgt.

15. Verfahren nach einem der Ansprüche 10 bis 14,
wobei das Erwärmen des Reaktionsgemisches in Verfahrensschritt d) auf eine Temperatur in dem Bereich von 80 °C bis 160 °C erfolgt.
